# EUROPEAN PATENT APPLICATION

(11) **EP 2 821 076 A1**
(43) Date of publication of application: **07.01.2015**
(21) Application number: 13305940.2
(22) Date of filing: 02.07.2013
(51) Int. Cl.: A61K 38/17, A61P 11/06, A61P 37/08

(54) **C1q as a therapeutic agent of allergy and/or asthma**

(71) Applicant: STALLERGENES SA, 92160 Antony Cedex (FR)
(72) Inventor: Mascarell, Laurent, 75020 PARIS (FR); Moingeon, Philippe, 91370 VERRIERES LE BUISSON (FR); Airouche, Sabi, 75010 PARIS (FR); Bodo, Véronique, 91120 PALAISEAU (FR)
(74) Representative: Blot, Philippe Robert Emile

(57) **Abstract**

The present invention concerns C1q for use for treating allergy and/or asthma. The invention also relates to a pharmaceutical composition comprising C1q and at least one allergen and to products comprising C1q and at least one allergen as a combined preparation for use for treating allergy and/or asthma.

## Description

The present invention concerns C1q for use for treating allergy and/or asthma. The invention also relates to a pharmaceutical composition comprising C1q and at least one allergen and to products comprising C1q and at least one allergen as a combined preparation for use for treating allergy and/or asthma.

The human complement component C1q (C1q) is the recognition component of the classical pathway of complement activation. The best-known ligands for C1q are the Fc regions of aggregated immunoglobulin (Ig)G and IgM molecules in immune complexes. Such binding triggers activation of the classical pathway, one of the three mechanisms for the activation of complement system. The result of activation of the complement system is the generation of C3 and C5 convertases that generate pro-inflammatory C3a and C5a anaphylatoxins and catalyse formation of pore-like membrane attack complex that inserts into cells membranes and cause damages by lytic or sublytic mechanisms.

Besides its well-known role in complement activation, C1q is implicated in the biology of antigen presenting cells such as monocytes, macrophages and dendritic cells (DCs) which express or secrete this molecule (Lu et al. Cell Mol Immunol 5, 9-21, 2008). *In vitro* treatment of monocytes by C1q regulates cell differentiation and confers a tolerogenic phenotype to monocyte-derived DCs (Castellano et al. Eur. J. Immunol. 37, 2803-2811, 2007; Fraser et al. J. Immunol. 183, 6175-6185 2009). In contrast, C1q deficiency impairs the recognition and clearance of apoptotic cells which leads to the development of autoimmunity (e.g. Systemic lupus erythematosus (SLE), glomerulonephritis) (Botto et al. Nat. Genet. 19, 56-59, 1998; Nauta et al. Eur. Immunol. 32, 1726-1736, 2002).

Dendritic cells are specialized antigen presenting cells that integrate a variety of incoming signals to orchestrate adaptive immune responses.

These cells have peculiar and opposite abilities, and therefore can be distinguished in two major and differently specialized subpopulations: on the one hand the effector DCs (also called pro-inflammatory DCs) and on the other hand the tolerogenic DCs (also called regulatory or DCreg).

The effector DCs, when activated, are crucial for the presentation of peptides and proteins to T and B lymphocytes and are widely recognized as professional antigen-presenting cells (APC), thanks to their ability to prime naïve T cells.

This subpopulation is involved in responses against infectious pathogens and tumors. Depending on the type of pathogen or antigen encountered and the profile of co-stimulatory molecules engaged, effector DCs have the capacity to induce different polarizations of T helper lymphocytes, that is to drive the development of Th1, Th2 or Th17 effector CD4+ T cells.

The effector DC subpopulation can be divided into at least three distinct cell subsets regarding the helper T cells they are able to prime: DC1 cell subset which drives the development of Th1 cells (cells producing type 1 cytokines IFN-γ and IL-2), DC2 cell subset which drives the development of Th2 cells (cells producing type 2 cytokines IL-4, IL-5 and IL-13), and DC17 cell subset which drives the development of Th17 cells (cells producing IL-17).

In contrast, tolerogenic DCs mediate the suppression of antigen (Ag)-specific immune responses via the induction of regulatory (also called suppressive) CD4+ T cells, T-cell anergy and clonal deletion of T-cells. Tolerogenic DCs are thus critically involved in promoting and maintaining clinical and/or immunological tolerance, as well as regulating excessive and undesired immune responses. Regulatory/tolerogenic DCs have been shown to suppress inflammatory response to inhaled allergens (Swiecki and Colonna, Eur. J. Immunol., 40:2094-2098, 2010; Kuipers, Vaccine, 23(37):4577-4588, 2005; Lambrecht, Allergy, 60(3): 271-282, 2005).

Therefore, bidirectional interactions between DCs and T cells initiate either effector or tolerogenic responses, which are crucial to establish appropriate defence mechanisms, while precluding uncontrolled inflammation and immune response.

The Applicant recently showed in a pollen chamber study that induction of C1q expression in DCs correlates with clinical efficacy induced by allergenic immunotherapy (Zimmer et al. J. Allergy Clin. Immunol. 129, 1020-1030, 2012; international patent application WO 2013/034569).

The Applicant has now demonstrated that C1q promotes tolerance induction *in vivo* in a murine Th2-driven asthma model. Particularly, in ovalbumin (OVA) sensitized mice, the effect of C1q was studied with a dose range experiment (i.e. 10 µg, 50 µg and 100 µg) by assessing airway hyper-responsiveness (AHR), inflammatory cell infiltration (i.e. eosinophils and type 2 innate lymphoid cells (ILC2)) in broncho-alveolar fluids, Th2 cytokine production by OVA-specific lung T cells and seric OVA-specific IgE production. Except for OVA-specific IgE levels, all the parameters tested were significantly decreased in a dose dependent manner following C1q therapy.

The optimal (i.e. 50 µg) dose of C1q, in its native and heat-denatured forms, was further compared with an effective regimen (i.e. 60 µg) of dexamethasone (DEX), the gold standard for treating various acute and chronic inflammatory diseases. Thereby, the applicant demonstrated that native C1q, but not heat-denatured C1q, was as efficient as DEX to treat allergic asthma using readouts as described above.

Taken together the results identify C1q as a molecule for treating allergy and/or asthma.

### Definitions

"C1q" denotes the first subcomponent of the C1 complex of the classical pathway of complement activation. Throughout the specification, the terms "C1q" and "C1Q" are used indistinctively.

In human, C1q is composed of 18 polypeptide chains: six C1qA chains (UniProt/Swiss-Prot accession number C1QA_HUMAN, SEQ ID NO:1 or any polymorphic variant thereof; mature form of 223 amino acids spanning positions 23-245 of SEQ ID NO:1), six C1qB chains (UniProt/Swiss-Prot accession number C1QB_HUMAN, SEQ ID NO:2 or any polymorphic variant thereof; mature form of 226 amino acids spanning positions 28-253 of SEQ ID NO:2), and six C1qC chains (UniProt/Swiss-Prot accession number C1QC_HUMAN, SEQ ID NO:3 or any polymorphic variant thereof, mature form of 217 amino acids spanning positions 29-245 of SEQ ID NO:3). The A, B and C chains associate as six hetero-trimers to form the mature functional C1q complex. Each C1q chain contains an N-terminal collagen-like domain and a C-terminal globular domain (gC1q). The majority of C1 complex ligands bind to the globular "recognition" domains of C1q. In particular, the globular domains of C1q form the recognition binding sites that interact with the exposed CH2 domains in the Fc regions of aggregated IgG and IgM in immune complexes. However, C1q does not bind to IgA, IgE or IgD immune complexes (Sontheimer et al., J. Invest. Dermatol. 125:14-23, 2005).

C1qA, C1qB and/or C1qC chains may comprise post-translational modifications, as compared with the sequences shown in SEQ ID NO:1-3, respectively.

For instance, C1qA (as shown in SEQ ID NO:1) may comprise one or more of the following amino acid modifications: 5-hydroxylysine at position(s) 33, 48, 67, 100 and/or 103, and/or 4-hydroxyproline at position(s) 39, 45, 54, 57, 73, 85, and/or 97, and/or O-linked (Gal) at position(s) 33, 38, 67, 100, and/or 103, and/or N-linked (GIcNAc) at position 146 of the pre-protein.

For instance, C1qB (as shown in SEQ ID NO:2) may comprise the following amino acid modification: Pyrrolidone carboxylic acid at position 28 of the pre-protein.

For instance, C1qC (as shown in SEQ ID NO:3) may comprise one or more of the following amino acid modifications: 4-hydroxyproline at position(s) 36, 39, 42, 45, 54, 63, 81, 93, 96, 99, and/or 105, and/or 5-hydroxylysine at position(s) 57 and/or 75, and/or O-linked (Gal) at position 75 of the pre-protein.

C1q may denote human or non-human mammalian C1q, in particular rat, mouse, cat, dog or monkey C1q.

As used in the instant application, the term "C1q" denotes the soluble C1q complex (i.e. C1q complex in free form) as well as polymerised C1q complex, or biologically active fragments thereof.

As used herein, "polymerised C1q complex" denotes covalently or non-covalently bound C1q complexes, C1q complexes bound onto solid surfaces or C1q multimers.

In the context of the invention, "biologically active" denotes the capacity to display, induce or stimulate one or more of (i) anti-inflammatory and/or immunosuppressant activity, (ii) reduction of inflammatory cell recruitment, in particular eosinophils and/or type 2 innate lymphoid cells, or (iii) decreased Th2 cytokine expression by T cells.

A "Th2 cytokine" denotes IL-4, IL-5 and/or IL-13. Other cytokines are usually designated as "Th1 cytokines" (e.g. IFN-γ and IL-2) or "Th17 cytokines" (e.g. IL17 and IL23).

"Type 2 innate lymphoid cells" or "ILC2s" denote side scatter (SSC) low, lineage negative cells expressing ICOS as well as the IL-33 receptor T1/ST2, i.e. SSC^{low} Lin⁻ICOS⁺ T1/ST2⁺ cells.

The term "treating" or "treatment", as used herein, means reversing, alleviating, inhibiting the progress of, or preventing the disorder or condition to which such term applies, or one or more symptoms of such disorder or condition.

A "subject" denotes a human or non-human mammal, in particular a rodent, a feline, a canine or a primate. Preferably, a subject denotes a human, in particular a child, a woman, a man.

As used herein, the word "comprising" is to be interpreted as encompassing all the specifically mentioned features of the claim, as well optional, additional, unspecified ones; The word "comprising" also discloses the embodiment in which only those features as specified in the claim are present (i.e. "consisting of").

### C1q therapeutic indication

The Applicant showed *in vivo,* in an OVA-sensitized murine model of asthma, that C1q:
- decreased airway hyper-responsiveness upon aerosol challenge to OVA,
- decreased inflammatory cell infiltration in the broncho-alveolar fluids, in particular eosinophils and ILC2 infiltration,
- decreased pro-allergenic Th2 cytokine production by OVA-specific lung T cells and
- was as efficient as DEX to treat allergic asthma using the readouts as described above.

The observed effects of C1q on the inflammatory response in this model of asthma, together with the fact that C1q mimicked the activity of dexamethasone, one of the most potent anti-inflammatory and immunosuppressive molecules, identify C1q as an anti-inflammatory and/or immunosuppressant agent for the treatment of allergy and/or asthma.

The invention thus relates to C1q for use for treating allergy and/or asthma.

An "allergen" is a substance, usually a protein, which elicits the production of IgE antibodies in predisposed individuals. Allergens may include pollen allergens (such as tree, herb, weed and grass pollen allergens), insect allergens (such as inhalant, saliva and venom allergens, e.g. cockroach, midge and house dust mite allergens and hymenoptera venom allergens), animal hair and dander allergens (from e.g. dog, cat, horse, rat, mouse, rabbit) and food allergens.

For instance, a protein allergen may be selected from the group consisting of a protein allergen of the genus Dermatophagoides; a protein allergen of the genus Felis; a protein allergen of the genus Ambrosia; a protein allergen of the genus Lolium; a protein allergen of the genus Cryptomeria; a protein allergen of the genus Alternaria; a protein allergen of the genus Alder, a protein allergen of the genus Betula; a protein allergen of the genus of Blomia; a protein allergen of the genus Quercus; a protein allergen of the genus Olea; a protein allergen of the genus Artemisia; a protein allergen of the genus Plantago; a protein allergen of the genus Parietaria; a protein allergen of the genus Canine; a protein allergen of the genus Blattella; a protein allergen of the genus Apis; a protein allergen of the genus Cupressus; a protein allergen of the genus Thuya; a protein allergen of the genus Chamaecyparis; a protein allergen of the genus Periplaneta; a protein allergen of the genus Agropyron; a protein allergen of the genus Secale; a protein allergen of the genus Triticum; a protein allergen of the genus Cynorhodon ; a protein allergen of the genus Juniperus ; a protein allergen of the genus Dactylis; a protein allergen of the genus Festuca; a protein allergen of the genus Poa; a protein allergen of the genus Lolium; a protein allergen of the genus Avena; a protein allergen of the genus Holcus; a protein allergen of the genus Anthoxanthum; a protein allergen of the genus Arrhenatherum; a protein allergen of the genus Agrostis; a protein allergen of the genus Phleum; a protein allergen of the genus Phalaris; a protein allergen of the genus Paspalum; and a protein allergen of the genus Sorghum.

Examples of various known protein allergens derived from some of the above-identified genus include : Betula (verrucosa) Bet v I ; Bet v II ; Blomia Blo 1 1; Blo t III; Blo t V; Blo t XII; Cynorhodon Cyn d I; Dermatophagoides (pteronyssinus or farinae) Der p I; Der p II; Der p III; Der p VII; Der f I; Der f II; Der f III; Der f VII; Felis (domesticus) Fel d I; Ambrosia (artemiisfolia) Amb a 1.1 ; Amb a 1.2; Amb a 1.3; Amb a 1.4; Amb a II; Lollium (perenne) Lol p I; Lot p II; Lol p III; Lot p IV; Lol p IX (Lol p V or Lol p Ib); Cryptomeria (japonica) Cry j I; Cry j II; Canis (familiaris) Can f I; Can f II; Juniperus (sabinoides or virginiana) Jun s I; Jun v I; Juniperus (ashei) Jun a I; Jun a II; Dactylis (glomerata) Dae g I; Dae g V; Poa (pratensis) Poa p I; Phl p I; Phl p V; Phl p VI and Sorghum (halepensis) Sor h I.

"Allergy" is a condition characterized by production of allergen-specific IgE in response to a specific allergen, usually a protein. Allergy denotes in particular "immediate allergy" also called "type I hypersensitivity". In type 1 hypersensitivity, an allergen is presented to CD4+ Th2 cells specific to the allergen that stimulate B-cell production of IgE antibodies also specific to the allergen.

Clinical manifestations and symptoms of allergy may include nasal congestion, nasal pruritis, ocular pruritis, tearing, rhinorrhoea, sinusitis, rhinitis, sneezing, wheezing, asthma, conjunctivitis, systemic anaphylaxis, localized anaphylaxis (atopy), atopic dermatitis, eczema, and mastocytosis induced anaphylactic shock.

"Asthma" is a common chronic inflammatory disease of the airways characterized by variable and recurring symptoms, reversible airflow obstruction, and bronchospasm. Common symptoms include wheezing, coughing, chest tightness, and shortness of breath.

In particular, in the method or use according to the invention, C1q has anti-inflammatory and/or immunosuppressant activity.

According to an embodiment, C1q reduces inflammatory cell recruitment, in particular recruitment of eosinophils and/or type 2 innate lymphoid cells (ILC2s).

Type 2 innate lymphoid cells play a key role in type 2 immune responses by prompt production of type 2 cytokines (especially IL-5 and IL-13) in response to antigen-induced IL-25/33. Accumulating evidences tend to indicate that ILC2s are mediators of type 2 pathologies such as allergy and asthma.

Furthermore, C1q decreases Th2 cytokine expression by allergen-specific T cells, in particular IL-5 and IL-13 expression.

Accordingly, C1q preferably decreases Th2 cytokine expression by T cells specific for said allergen and/or reduces recruitment of type 2 innate lymphoid cells.

According to an embodiment, C1q reduces airway hyper-responsiveness and/or bronchospasm when the disease is asthma.

C1q may be advantageously administered in combination (simultaneously, separately, or sequentially) with the allergen associated with the allergen-induced inflammatory response which is the hallmark of allergy and asthma. Without wishing to be bound by this theory, it is thought that C1q, when administered in combination with the allergen, could act as an adjuvant and stimulate induction of tolerance to the allergen.

### Pharmaceutical compositions

C1q is advantageously formulated in a pharmaceutical composition in order to be used in the medical indication or method of therapeutic treatment defined above.

The invention thus relates to a pharmaceutical composition comprising C1q for use for treating allergy and/or asthma.

The invention further relates to a pharmaceutical composition comprising C1q and at least one allergen.

The invention also relates to products comprising C1q and at least one allergen as a combined preparation for simultaneous, separate or sequential use for treating allergy and/or asthma induced by said at least one allergen. Formulation of C1q and said at least one allergen in separate products may indeed be appropriate in particular if a same route of administration in not adapted to administrate both C1q and said at least one allergen.

Said pharmaceutical composition or products comprising C1q and at least one allergen is(are) particularly intended for use for treating allergy and/or asthma induced by said at least one allergen, or to be administered to a subject suffering from allergy and/or asthma induced by said at least one allergen.

According to an embodiment said pharmaceutical composition or products has(have) anti-inflammatory and/or immunosuppressant activity.

More specifically, said pharmaceutical composition or products:
- reduce(s) inflammatory cell recruitment, in particular recruitment of eosinophils and/or type 2 innate lymphoid cells (ILC2s), and/or
- decrease(s) Th2 cytokine expression by allergen-specific T cells, in particular IL-5 and IL-13 expression, and/or
- reduces airway hyper-responsiveness and/or bronchospasm when the disease is asthma.

Accordingly, the pharmaceutical composition or products preferably decrease Th2 cytokine expression by T cells specific for said allergen and/or reduces recruitment of type 2 innate lymphoid cells.

The pharmaceutical composition or products also reduce(s) airway hyper-responsiveness and/or bronchospasm when the disease is asthma.

The pharmaceutical composition or products comprise(s) with a pharmaceutically acceptable excipient, in addition to C1q and said at least one allergen.

"Pharmaceutically acceptable" means it is, within the scope of sound medical judgment, suitable for use in contact with the cells of humans and lower animals without undue toxicity, irritation, allergic response and the like, and are commensurate with a reasonable benefit/risk ratio.

As used herein, the term "pharmaceutically acceptable excipient" includes solvents, dispersion media, coatings, antibacterial and antifungal agents, isotonic and absorption delaying agents, mucoadhesive excipients, and the like, that do not produce an adverse or other untoward reaction when administered to an animal, or a human, as appropriate. Excipients may further include, but are not limited to disintegrants, binders, lubricants, flavoring, colorants, preservatives.

Preferably, C1q is intended for administration by parenteral route, i.e. C1q is formulated in a composition suitable for parenteral administration.

Preferably, said at least one allergen is intended for administration by oromucosal route, still preferably by sublingual administration.

Where mucosal administration is contemplated, the pharmaceutically acceptable excipient may advantageously be a "mucoadhesive carrier". As intended herein, a "mucoadhesive carrier" enables close and prolonged contact with a mucosa, in particular a mucosa of the oral cavity, and more particularly the sublingual mucosa, thereby enhancing-antigen (allergen) specific tolerance induction. Preferred mucoadhesive carriers as defined herein notably comprise chitosan, polymers of maltodextrin or carboxymethylcellulose.

For parenteral administration in an aqueous solution, for example, the solution should be suitably buffered if necessary and the liquid diluent first rendered isotonic with sufficient saline or glucose. These particular aqueous solutions are especially suitable for intramuscular and subcutaneous administration. In this connection, sterile aqueous media which can be employed will be known to those of skill in the art in light of the present disclosure.

The pharmaceutical compositions or the products according to the invention can include any conventional adjuvant. For oromucosal administration, the adjuvants may preferably be a Bifidobacterium, a lactic acid bacterium (either in the form of a cell suspension, freeze-dried cells, a lysate, purified sub-components, or purified molecules), or a combination of a corticosteroid with vitamin D3 or any metabolite or analog of the latter. Preferably, the pharmaceutical composition, or the products is(are) to be administered by the mucosal route, more preferably by the oromucosal route, and most preferably by the sublingual route.

The medicaments according to the invention can be administered in various forms, such as dispersed forms, e.g. in suspensions or gels, or as dry forms, e.g. in powders, tablets, capsules, delayed release capsules, lyoc, or forms suitable to be administered in a metered-dosing device. The use of liposomes and/or microparticles and/or nanoparticles is also possible. The use and formation of liposomes and/or microparticles and/or nanoparticles are known to those skilled in the art.

In the frame of methods for treating allergy or asthma with the pharmaceutical composition or the products according to the invention, the administration regimen may be maintained for instance for a period of less than 6 weeks to more than 3 years.

In particular, C1q is present in a therapeutically effective amount in said pharmaceutical compositions or product of the combined preparation.

Mammalian C1q, and in particular human C1q, can be readily purified from plasma, for instance by a method as described in the international patent application WO 2010/094901, to prepare such pharmaceutical compositions and products.

Some variation in dosage will necessarily occur depending on the condition of the subject being treated. Dosages to be administered depend on individual needs, on the desired effect and the chosen route of administration. It is understood that the dosage administered will be dependent upon the age, sex, health, and weight of the recipient, concurrent treatment, if any, frequency of treatment, and the nature of the effect desired. The total dose required for each treatment may be administered by multiple doses or in a single dose. The person responsible for administration will, in any event, determine the appropriate dose for the individual subject.

The invention will be further illustrated by the following figures and examples.

### FIGURES

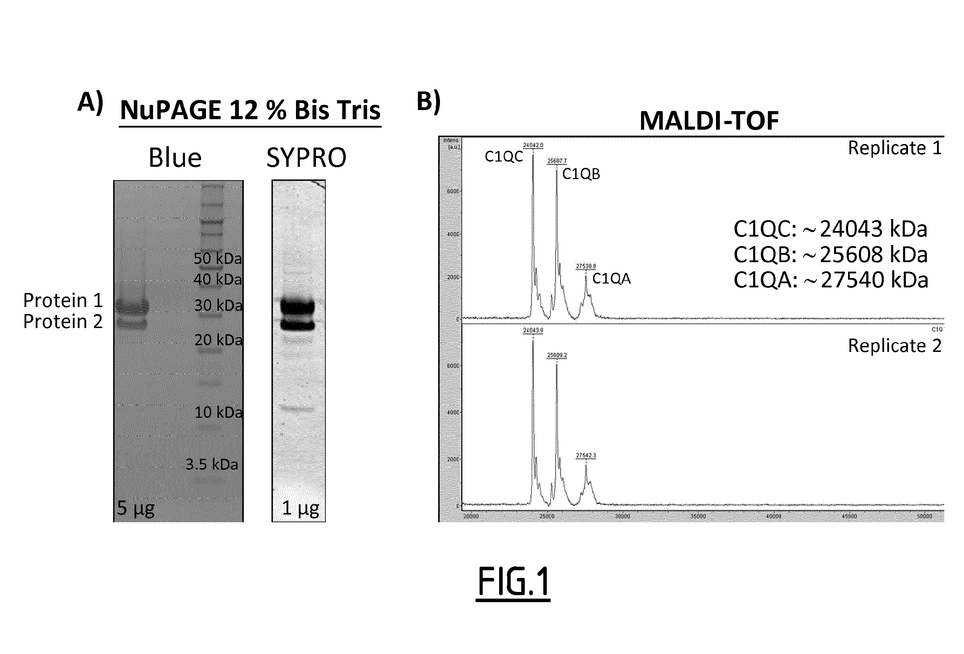

Figure 1: A: 1D-gel electrophoresis of human C1q, under reduced, denaturing conditions. B: Representative mass spectrometry spectra of human C1q.

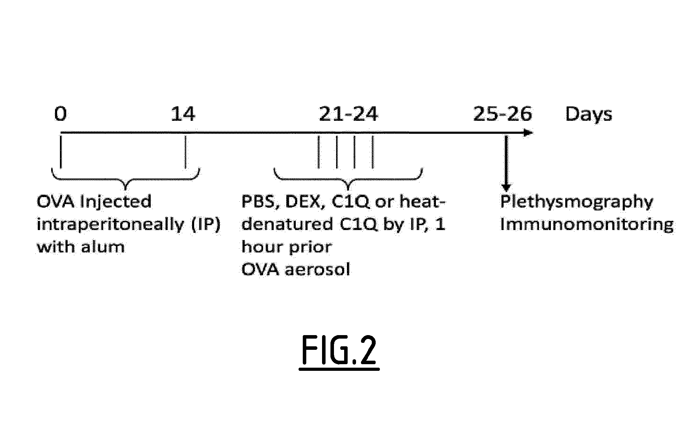

Figure 2: Experimental design. BALB/c mice were sensitized by intra-peritoneal (i.p.) injections with OVA/Alum (days 0 and 14) followed by aerosol challenges (days 21-24) with OVA. BALB/c mice were intraperitoneally treated one hour before each aerosol challenge with either PBS, C1q (10-100 µg/dose), heat-denatured C1q (50 µg/dose) or DEX (60 µg/dose). AHR measurements and immunomonitoring were performed at days 25 and 26, respectively.

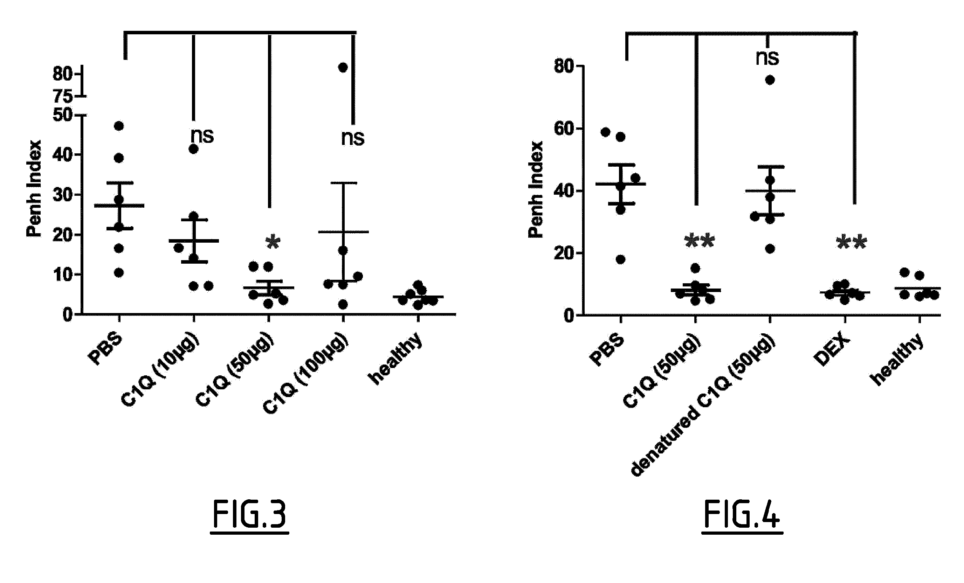

Figures 3-4: C1q therapy significantly decreased airway hyperresponsiveness in a dose dependent manner. Airway responsiveness was determined by measuring the Penh value in response to metacholine. Horizontal bars represent the mean response +/- SEM within each group with each dot representing the Penh value obtained in an invididual animal. n = 6 mice for PBS, C1q (10-100 µg/dose), heat-denatured C1q (50 µg/dose) or DEX (60 µg/dose) treated mice. n = 7 for healthy mice in figure 3 and n = 6 for healthy mice in figure 4. ns = non-significant, * p < 0.05 and ** p < 0.01 compared with mice treated with PBS. Data were compared using the nonparametric Kruskal-wallis test.

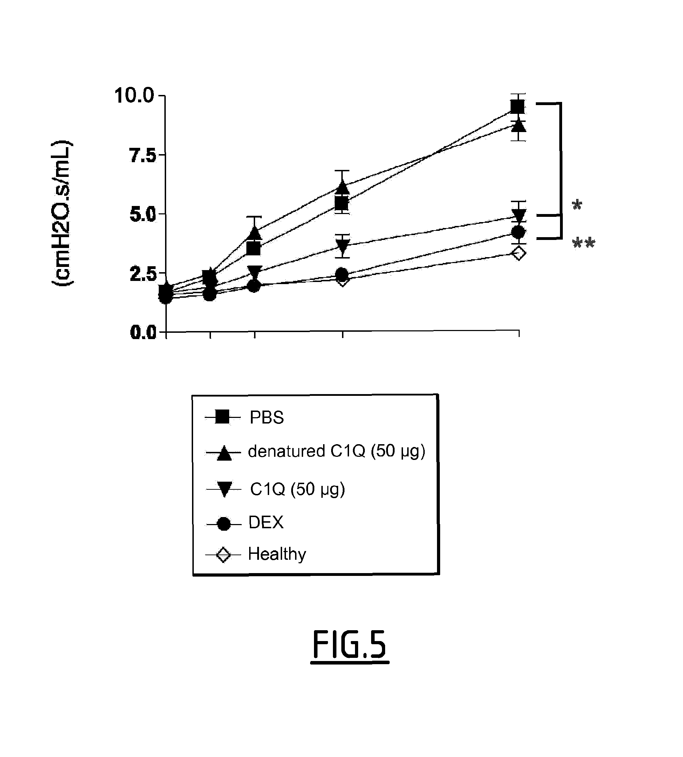

Figure 5: C1q treatment significantly reduced airway resistance. Bronchial resistance was measured using a FinePointe RC system 24 hrs after the last aerosol. Results are expressed as mean values ± SEM, within each group. n = 6 mice for PBS, C1q (50 µg/dose), heat-denatured C1q (50 µg/dose) or DEX (60 µg/dose) treated mice. ns = non-significant, * p < 0.05 and ** p < 0.01 compared with mice treated with PBS. Data were compared using the nonparametric Kruskal-Wallis test.

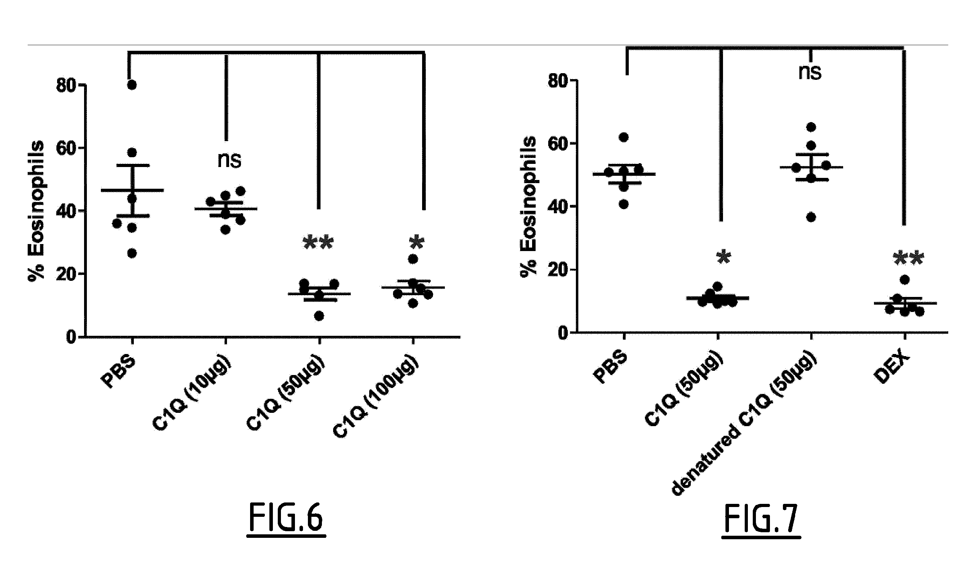

Figures 6-7: C1q therapy significantly inhibited the OVA-induced eosinophil infiltration in broncho-alveolar lavages (BAL) fluid. Eosinophils were counted in BALs from mice receiving the various treatments. Results are shown as (mean +/- SEM). n = 5-6 mice for PBS, C1q (10-100 µg/dose), heat-denatured C1q (50 µg/dose) or DEX (60 µg/dose) treated mice. ns = non-significant, * p < 0.05 and ** p < 0.01 compared with mice treated with PBS. Data were compared using the nonparametric Kruskal-Wallis test.

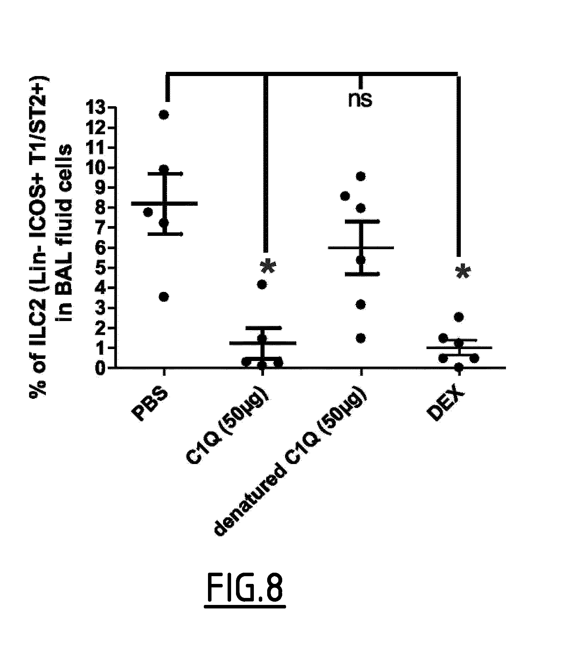

Figure 8: C1q therapy significantly decreased the OVA-induced ILC2s infiltrates in BALs. The percentage of ILC2 (SSC^{low}Lin⁻ICOS⁺T1/ST2⁺) was analyzed in BAL fluid from mice receiving the various treatments. Results are shown as (mean +/- SEM). n = 5-6 mice for PBS, C1q (50 µg/dose), heat-denatured C1q (50 µg/dose) or DEX (60 µg/dose) treated mice. ns = non-significant and * p < 0.05 compared with mice treated with PBS. Data were compared using the nonparametric Kruskal-Wallis test.

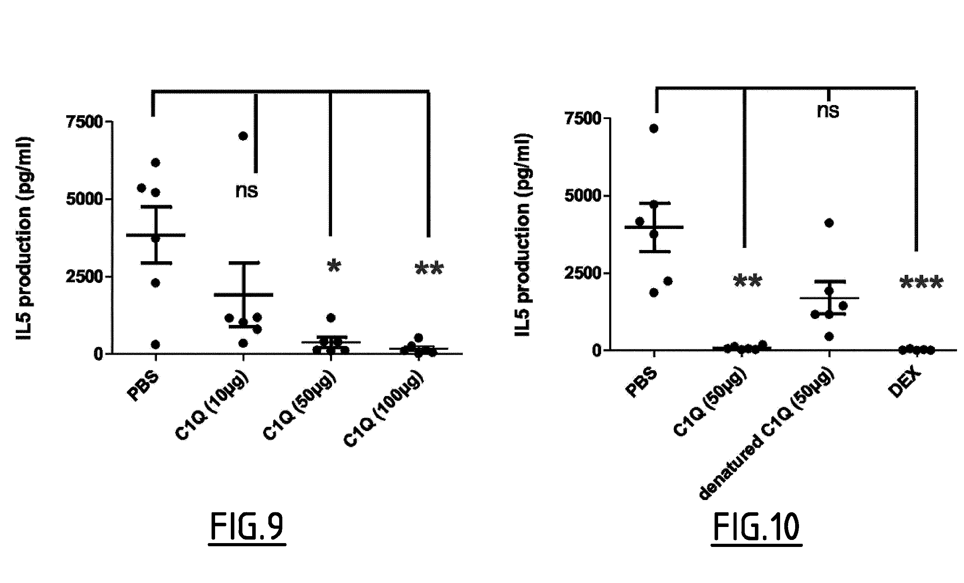

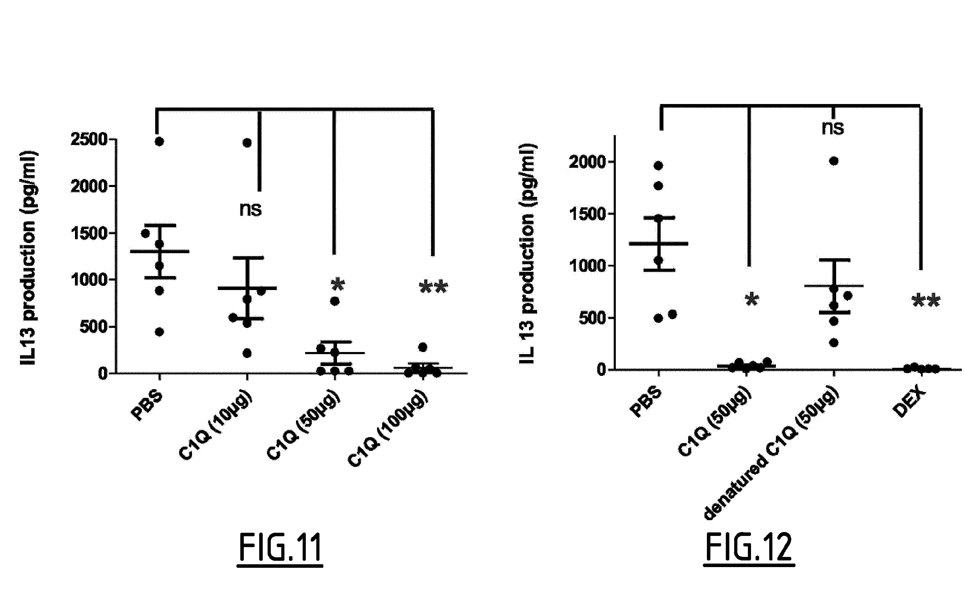

Figures 9-12: C1q treatment significantly reduced Th2 responses in the lungs. Levels of IL5 and IL13 were assessed in culture supernatants of OVA-stimulated lung T cells using a Mouse Cytokine Bead kit. Results are shown as (mean +/- SEM). n = 6 mice for PBS, C1q (10-100 µg/dose), heat-denatured C1q (50 µg/dose) or DEX (60 µg/dose) treated mice. ns = non-significant, * p < 0.05 and ** p < 0.01 compared with mice treated with PBS. Data were compared using the nonparametric Kruskal-Wallis test.

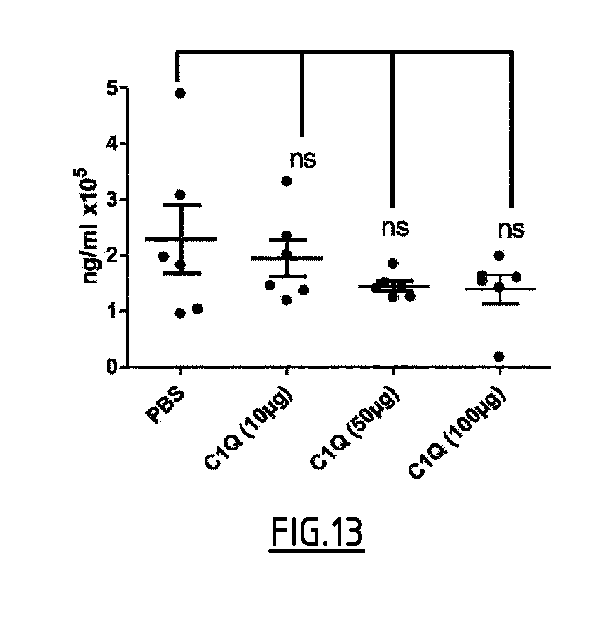

Figure 13: Therapeutic administration of C1q did not decrease OVA-specific IgE production. Mice were treated as described in methods. Levels of seric OVA -specific IgE were measured by ELISA as described in methods. Ns = non-significant compared with mice treated with PBS. Data were compared using the nonparametric Kruskal-Wallis test .

### EXAMPLES

### EXAMPLE 1: Characterization of C1q

Human C1q, purified from serum, was obtained from Calbiochem (#204876).

### 1D-gel electrophoresis

Protein sample was fractionated by 1D-gel electrophoresis (#204876 Complement C1q, Human, Calbiochem, 1.1 µg/µl). NuPAGE® LDS sample buffer and NuPAGE® reducing agent were used to prepare protein samples for denaturing gel electrophoresis with the NuPAGE® gels (Life Technologies). Proteins were separated by NuPAGE® Bis-Tris gel 4-12% with MES running buffer (1 and 5 µg/lane) and stained with Sypro Ruby and Coomassie® G-250 StainSimplyBlue (Life Technologies), respectively. Novex® sharp unstained protein standard was used to estimate molecular masses over a large range. Representative gel images are shown in Figure 1A. Protein bands were then excised from gels (automated Bio-Rad spot picker), processed by tryptic in-gel digestion and analyzed by nLC-MS/MS.

### nLC-MS/MS

Tryptic peptides samples (*in gel* or *in solution* digestions respectively) were separated by reverse-phase chromatography using an Ultimate 3000 RS nano LC system (Thermo scientific). The nanoHPLC was coupled to an ESI-Qq-TOF mass spectrometer (Maxis, Bruker Daltonics). Peptides were loaded for 10 min onto the Acclaim PepMap100 column (100 µm x 2 cm; C18, 5 µm, 100 Ǻ, Thermo scientific) with a flow rate of 12 µL/min and buffer A (2% ACN, 0.15% FA). Separation was then performed using an Acclaim PepMap RSLC column (75 µm x 15cm; C18, 2 µm, 100Ǻ, Thermo scientific) with a flow rate of 450 nL/min. For accurate mass measurements, the lock mass option was enabled in MS mode: m/z 299.2945 (methylstearate, Sigma and m/z 1221.9906 (Chip cube high mass reference, Agilent) ions generated in the electrospray process from ambient air were used for internal recalibration. Internal recalibration was performed using Data Analysis software. NanoLC-MS/MS data were analyzed using an in-house Mascot server (Matrix Science, version 2.3) to search Uniprot/Swiss-Prot databases, assuming tryptic digestion. Precursor mass and fragment mass were searched with initial mass tolerance of 8 ppm and 0.05 Da, respectively. The search included fixed modification of carbamidomethyl cysteine. Minimal peptide length was set to 6 amino acids and a maximum of one miscleavage was allowed. Peptide identifications were accepted if they could be established at a greater than 95% probability as specified by Mascot software.

### MALDI-MS

Protein sample (#204876 Complement C1q, Human, Calbiochem,1.1 µg/µl) was reduced with TCEP 25 mM (5 min, RT) and then loaded onto a StageTips C8 and desalted with 0.1% TFA. Proteins were directly eluted from the StageTips on the MALDI plate using 2 µL of Sinapinic acid matrix at 12 mg/mL in 50 % acetonitrile, 0.1% TFA. Protein analyses were carried out on a MALDI TOF/TOF Autoflex Speed mass spectrometer (Bruker Daltonics) equipped with the smartbeamTM-II laser technology. Spectra were obtained in linear mode by accumulating an average of ∼ 2500 shots at a 1 KHz repetition rate. The calibration was performed externally in positive mode using the protein calibration standard II mixture (Bruker Daltonics). Data were processed with FlexAnalysis 3.3 software (Bruker Daltonics). Representative MS spectra are shown in Figure 1B.

### Results

1D-gel/nLC-MS/MS analysis revealed two protein bands corresponding to C1QA/C1QB and C1QC, respectively (Figure 1A). Trace amounts of other proteins were slightly detected with SYPRO Ruby dye. The MALDI-MS analysis of the reduced C1Q revealed the expected A, B and C chains (Figure 1B, Tissot et al., Biochemistry 2005, 44, 2602-2609). Finally, sample was also digested *in solution* with trypsin, and only C1 QA/B/C subunits were successfully identified by mass spectrometry, showing a high level of purity (data not shown).

### EXAMPLE 2: The secondary structure of C1q is irreversibly abolished after heat-incubation

In order to compare biologically active and inactive forms of C1q in a murine asthma model, C1q thermal stability was first evaluated by performing a CD temperature-dependent study that measures secondary structure unfolding while temperature is increased.

### Circular dichroism (CD) spectroscopy

C1q was obtained from Cabiochem® (#204876 Complement C1q, Human, Calbiochem).

The CD spectra were recorded with a six-cell Peltier temperature-controlled Jasco-815 spectropolarimeter. C1q concentration was 1.14 mg/mL, and 40 µL of sample (approximately 46 µg of protein) were loaded in 0.1 mm path length cells. The buffer consisted of 10 mM HEPES, 300 mM NaCl and 40% glycerol v/v (pH 7.2). A CD measurement control was performed at wavelength ranging from 190 to 260 nm. The temperature-dependent circular dichroism was monitored at temperatures ranging from 30°C to 90°C at a wavelength 200 nm (2.5°C/min). CD Spectrum was smoothed using noise reduction.

### Results

The secondary structure of C1q was stable until 54°C and completely denatured at 84°C. The native secondary structure of C1q was not recovered following heat denaturation at 90°C, then cooling down at 20°C, demonstrating that C1q secondary structure was irreversibly altered.

Thus, in the experiments described below, C1q was incubated at 84°C for 10 min. resulting in an irreversible denaturation of the protein.

### EXAMPLE 3: The human complement component C1q promotes tolerance in asthma and airway diseases

### 3.1 MATERIALS AND METHODS

### Mice, reagents and antibodies

Six- to eight-weeks-old BALB/c female mice were obtained from Charles River (L'Arbesle, France). Phosphate-buffer saline (PBS) was purchased from Invitrogen (Carlsbad, CA). OVA grade V with low endotoxin content was purchased from Sigma (St. Louis, MO) and was further purified on an endotoxin removing gel (Pierce, Rockford, IL). Residual endotoxin concentrations determined by Endochrome-K assay (R1708K, Charles River, Wilmington, MA) were always less than 0.1 EU/ µg protein. Human purified C1q was obtained from Calbiochem (#204876) (distributed by Merck (Darmstadt, Germany) and DEX was purchased from Sigma.

### Therapy model and measurements of airway inflammation in BALB/c mice

For sensitization, mice were immunized intraperitoneally (i.p.) on days 0 and 14 with 10µg OVA adsorbed on 2 mg Al(OH)3 (Pierce), administered in 100 µl PBS. From day 21 to 24, a daily 20 min aerosol challenge was performed with 1% w/v OVA using an aerosol delivery system (Buxco Europe Ltd, Winchester, UK). To test its potential tolerogenic activity, C1q (10-100 µg/dose) or heat-denatured C1q (50 µg/dose) was administered intraperitoneally one hour before each aerosol challenge. PBS and DEX (60 µg/dose) were administered as negative and positive controls, respectively. Measurements of AHR were performed by whole body plethysmography (Buxco) and results were expressed as enhanced pause (Penh). The Penh index, expressed as an increase relative to the baseline airway resistance, was obtained by dividing the Penh value measured after exposure to increased inhaled metacholine (from 0 to 50 mg) with the one measured after inhalation of nebulised PBS, as previously described (Razafindratsita et al., J Allergy Clin. Immunol. 120, 278-285 (2007)). We complemented those Penh measurements with invasive determination of resistance that directly measures pulmonary function. Briefly, mice anesthetized with ketamine/xylazine by i.p (100 mg/kg and 10 mg/kg, respectively, Centravet, Maisons-Alfort, France) were carefully intubated orotracheally. Animals were then placed in a plethysmograph and connected via the endotracheal cannula to a FinePointe RC system (Buxco). Inhalation exposure in orotracheally intubated animals was focused to the lungs, with no nasal nor oral intake. Bronchial resistance was measured using the FinePointe RC system after exposure to increasing doses (i.e. 1.875, 3.75, 7.5, 15 mg/ml) of methacholine using a protocol adapted from Swedin and al. (Int. Arch. Allergy Immunol. 153, 249-258 (2010)) .

For analysis of inflammatory cells in broncho-alveolar lavages (BAL), mice were anesthetized with pentobarbital/xylazine by i.p (50 mg/kg and 10mg/kg, respectively, Centravet, Maisons-Alfort, France), and BAL performed with 3 x 400 µl PBS. BAL fluid was centrifuged at 800 g for 10 min at 4°C. Cell pellets were resuspended in PBS, spun onto glass slides by cytocentrifugation, fixed and stained with May-Grünwald Giemsa (Réactifs RAL, Martillac, France). Eosinophils and macrophages were counted under light microscopy using a 200-fold magnification.

### Flow cytometry analysis (FACS) of type 2 innate lymphoid cells (ILC2) in BALs

To analyse the presence of inflammatory ILC2s in BAL fluids, cells were stained, at 4°C for 15 min, with monoclonal antibodies (mAbs) against CD4 (GK1.5), CD3 (clone 1452CM), CD8 (clone 53-6.7), CD11b (clone M1/70), CD19 (clone 1D3), CD11c (clone N418), FcεR1 (clone MAR-1), all conjugated to phycoerythrin (PE), T1/ST2 (clone DJ8) conjugated to fluorescein (FITC), ICOS (clone C398.4A) conjugated to allophycoerythrin. Corresponding isotype-matched mAbs were used as controls. All antibodies were purchased from e-Bioscience (San Diego, CA) except for mAbs against T1/ST2 from MD bioscience (St Paul, MN). The samples were acquired by using a FACSVerse (Becton Dickinson, Le pont de Claix, France) and analyzed with FlowJo software.

### Analysis of allergen-specific T cell responses in the lungs

To recover cells from lung tissues, one lobe was incubated for 1 h in RPMI supplemented with digest reagent (collagenase 75 U/mL; Roche, Basel, Switzerland). Isolated cells were filtered through a 70-µm sieve and washed twice before resuspension in culture medium. Lung cells were plated at 10⁶ cells per well and stimulated with OVA (100 µg/ml) or medium alone. After 72 hours at 37°C in 5% CO2 / 95% air, IL5 and IL13 were measured in culture supernatants using a Mouse Cytokine Bead kit (Merck Millipore, Darmstadt, Germany) and a Magpix system (Luminex, Austin, TX). Analyses were performed according to manufacturer's instruction.

### Measurement of allergen-specific IgE antibody responses

Sera were obtained after centrifugation of blood samples at 10 000 rpm for 10 min. For detection of OVA-specific IgE antibodies, were assessed in sera (at a 1/50 dilution) using the mouse ovalbumin specific IgE ELISA assay kit (AbD serotec, Düsseldorf, Germany) according to manufacturer's instructions. Optical densities were measured using an ELISA plate reader at 405 nm.

### Statistical analysis

Statistical differences between groups were assessed using a nonparametric test (Kruskal-Wallis). Results were considered statistically significant for a p-value below 0.05. Statistical and graphical analyses were performed using the Prism 5 software (GraphPad, La Jolla, CA).

### 3.2 RESULTS

### Design of immunotherapy in an OVA asthma mouse model

As described elsewhere (Razafindratsita et al. J Allergy Clin. Immunol. 120, 278-285 (2007)), mice sensitized with OVA using the protocol summarized in figure 1, develop AHR associated with elevated Penh values detectable by whole body plethysmography, as well as signs of lung inflammation with cellular infiltrates. The therapeutic effect of the human complement component C1q was tested in this in vivo murine model of established asthma as described in figure 2.

### C1q reduced airway hyperresponsiveness as well as lung inflammation in an OVA asthma mouse model

As expected, healthy (i.e. nonsensitized) mice exhibited low Penh values, whereas OVA-sensitized animals treated intraperitoneally with PBS displayed a high AHR (Figures 3 and 4). Intraperitoneal C1q treatment induced a dose dependent significant (p < 0.01) reduction of AHR when compared to PBS treated mice (Figures 3). Interestingly, administration of heat-denatured C1q had no impact on AHR (Figure 4). Also, OVA-sensitized mice intraperitoneally treated with DEX exhibited low Penh values (p < 0.01) as shown in figure 4.

Invasive monitoring of lung function in those animals confirmed as well a significant decrease in pulmonary resistance in groups receiving either C1q or DEX when compared to the PBS group (Figure 5; p < 0.05 and p < 0.01, respectively). Administration of heat-denatured C1q had no impact on pulmonary resistance when compared to PBS treated mice (Figure 5).

The decrease of AHR observed in C1q treated mice was associated with a significant dose dependent decrease (p < 0.01) in eosinophil counts in BALs when compared to the PBS group (Figure 6). A similar decrease was showed after DEX treatment (Figure 7; p < 0.01). As expected, administration of heat-denatured C1q had no effect on eosinophily (Figure 7).

Inflammatory ILC2s are also a part of lung infiltrating cells and dramatically increase in BAL fluid from OVA-sensitized mice (Barlow et al. J. Allergy Clin. Immunol. 129, 191-198 (2012)). ILC2s from BAL fluid are defined in flow cytometry as side scatter (SSC) low lineage negative cells expressing ICOS as well as the IL-33 receptor T1/ST2 (SSC^{low} Lin⁻ICOS⁺ T1/ST2⁺) (Barlow et al. J. Allergy Clin. Immunol. 129, 191-198 (2012)). A significant (p < 0.05) decrease in ILC2s was observed in both DEX and C1q treated mice when compared to PBS treated mice (Figure 8) whereas the percentage of ILC2s in BAL fluid from heat-denatured C1q treated mice was comparable to the one observed in the PBS group (Figure 8).

### C1q therapy significantly decreased Th2 responses in the lungs.

We further investigated whether administration of C1q altered cytokines produced in lungs by OVA-specific T cells re-stimulated by OVA in vitro. A significant (p < 0.01) decrease in IL-5 and IL-13 secretion by lung T cells was observed in both DEX and C1q treated mice when compared to PBS treated mice (Figures 9-12). Conversely, lung T cells from heat-denatured C1q treated mice secreted similar levels of IL-5 and IL-13 when compared to the PBS group (Figures 10 and 12). Interestingly, similar results were obtained in the spleen (data not shown).

### C1q therapy did not induce down-regulation of OVA-specific IgE antibodies

As expected, OVA-sensitized mice treated with PBS increased OVA-specific IgE antibodies. However, C1q had no significant impact on OVA-specific seric IgE antibodies (Figure 13).

### CONCL USION

Based on data obtained in 3 independent experiments, C1q acted similarly to dexamethasone, the gold standard of glucocorticoids, in counteracting OVA-induced airway hyperresponsiveness and recruitment of pro-inflammatory cells (i.e. eosinophils and type 2 innate lymphoid cells) in the lung. In addition, both C1q and DEX inhibited Th2 cytokine production in the lung. In conclusion, we clearly showed the anti-inflammatory effect of C1q in a Th2-driven asthma model, suggesting a potential therapeutic benefit of this molecule in humans.

SEQUENCE LISTING

<110>  Stallergenes S.A.

<120>  C1q as a therapeutic agent of allergy and/or asthma

<130>  BET13P1111

<160>  3

<170>  PatentIn version 3.5

<210>  1
<211>  245
<212>  PRT
<213>  Homo sapiens

<220>
<221>  SIGNAL
<222>  (1)..(22)

<220>
<221>  VARIANT
<222>  (23)..(23)
<223>  variant rs17887074 (E=>K)

<220>
<221>  mat_peptide
<222>  (23)..(245)

<220>
<221>  DOMAIN
<222>  (31)..(109)
<223>  collagen-like

<220>
<221>  DOMAIN
<222>  (110)..(245)
<223>  C1q

<220>
<221>  DOMAIN
<222>  (110)..(245)
<223>  C1qA globular domain

<400>  1

<210>  2
<211>  253
<212>  PRT
<213>  Homo sapiens

<220>
<221>  SIGNAL
<222>  (1)..(27)

<220>
<221>  mat_peptide
<222>  (28)..(253)

<220>
<221>  DOMAIN
<222>  (37)..(86)
<223>  Collagen-like 1

<220>
<221>  DOMAIN
<222>  (60)..(114)
<223>  Collagen-like 2

<220>
<221>  DOMAIN
<222>  (117)..(253)
<223>  C1qB globular domain

<400>  2

Met Met Met Lys Ile Pro Trp Gly Ser Ile Pro Val Leu Met Leu Leu
        -25                 -20                 -15

Leu Leu Leu Gly Leu Ile Asp Ile Ser Gln Ala Gln Leu Ser Cys Thr
    -10                 -5                  -1  1               5

Gly Pro Pro Ala Ile Pro Gly Ile Pro Gly Ile Pro Gly Thr Pro Gly
                10                  15                  20

Pro Asp Gly Gln Pro Gly Thr Pro Gly Ile Lys Gly Glu Lys Gly Leu
            25                  30                  35

Pro Gly Leu Ala Gly Asp His Gly Glu Phe Gly Glu Lys Gly Asp Pro
        40                  45                  50

Gly Ile Pro Gly Asn Pro Gly Lys Val Gly Pro Lys Gly Pro Met Gly
    55                  60                  65

Pro Lys Gly Gly Pro Gly Ala Pro Gly Ala Pro Gly Pro Lys Gly Glu
70                  75                  80                  85

Ser Gly Asp Tyr Lys Ala Thr Gln Lys Ile Ala Phe Ser Ala Thr Arg
                90                  95                  100

Thr Ile Asn Val Pro Leu Arg Arg Asp Gln Thr Ile Arg Phe Asp His
            105                 110                 115

Val Ile Thr Asn Met Asn Asn Asn Tyr Glu Pro Arg Ser Gly Lys Phe
        120                 125                 130

Thr Cys Lys Val Pro Gly Leu Tyr Tyr Phe Thr Tyr His Ala Ser Ser
    135                 140                 145

Arg Gly Asn Leu Cys Val Asn Leu Met Arg Gly Arg Glu Arg Ala Gln
150                 155                 160                 165

Lys Val Val Thr Phe Cys Asp Tyr Ala Tyr Asn Thr Phe Gln Val Thr
                170                 175                 180

Thr Gly Gly Met Val Leu Lys Leu Glu Gln Gly Glu Asn Val Phe Leu
            185                 190                 195

Gln Ala Thr Asp Lys Asn Ser Leu Leu Gly Met Glu Gly Ala Asn Ser
        200                 205                 210

Ile Phe Ser Gly Phe Leu Leu Phe Pro Asp Met Glu Ala
    215                 220                 225

<210>  3
<211>  245
<212>  PRT
<213>  Homo sapiens

<220>
<221>  SIGNAL
<222>  (1)..(28)

<220>
<221>  mat_peptide
<222>  (29)..(245)

<220>
<221>  DOMAIN
<222>  (31)..(112)
<223>  Collagen-like

<220>
<221>  DOMAIN
<222>  (115)..(245)
<223>  C1qC globular domain

<400>  3

## Claims

1. C1q for use for treating allergy and/or asthma.

2. C1q for the use according to claim 1, wherein C1q has anti-inflammatory and/or immunosuppressant activity.

3. C1q for the use according to claim 1 or 2, wherein C1q reduces inflammatory cell recruitment.

4. C1q for the use according to claim 3, wherein inflammatory cells are eosinophils and/or type 2 innate lymphoid cells.

5. C1q for the use according to any one of claims 1 to 4, wherein C1q decreases Th2 cytokine expression by T cells specific for said allergen associated with allergy and/or asthma, and/or C1q reduces recruitment of type 2 innate lymphoid cells.

6. C1q for the use according to any one of claims 1 to 5, wherein C1q reduces airway hyper-responsiveness and/or bronchospasm when the disease is asthma.

7. A pharmaceutical composition comprising C1q and at least one allergen.

8. A pharmaceutical composition comprising C1q and at least one allergen for use for treating allergy and/or asthma induced by said at least one allergen.

9. The pharmaceutical composition for the use according to claim 8, wherein said pharmaceutical composition has anti-inflammatory and/or immunosuppressant activity.

10. The pharmaceutical composition for the use according to claim 8 or 9, wherein said pharmaceutical composition reduces inflammatory cell recruitment.

11. The pharmaceutical composition for the use according to claim 10, wherein inflammatory cells are eosinophils and/or type 2 innate lymphoid cells.

12. The pharmaceutical composition for the use according to any one of claims 8 to 11, wherein said pharmaceutical composition decreases Th2 cytokine expression by T cells specific for said allergen and/or reduces recruitment of type 2 innate lymphoid cells.

13. The pharmaceutical composition for the use according to any one of claims 8 to 12, wherein said pharmaceutical composition reduces airway hyper-responsiveness and/or bronchospasm when the disease is asthma.

14. Products comprising C1q and at least one allergen as a combined preparation for simultaneous, separate or sequential use for treating allergy and/or asthma induced by said at least one allergen.

15. Products according to claim 14, wherein C1q is intended for administration by parenteral route and said at least one allergen is intended for administration by oromucosal route.
